# EUROPEAN PATENT APPLICATION

(11) **EP 1 733 737 A1**
(43) Date of publication of application: **20.12.2006**
(21) Application number: 05720517.1
(22) Date of filing: 10.03.2005
(51) Int. Cl.: A61K 45/00, A61K 31/4409, A61K 39/395, A61P 25/00, A61P 25/28

(54) **AXON REGENERATION PROMOTER**

(30) Priority: 11.03.2004 JP 2004068849; 21.09.2004 JP 2004273041
(71) Applicant: Bioclues, Inc., Tokyo 103-0007 (JP)
(72) Inventor: YAMASHITA, Toshihide c/o National University, Chuo-ku, Chiba 260-8670 (JP); HATA, Katsuhiko c/o National University, Chuo-ku, Chiba 260-8670 (JP)
(74) Representative: Vossius & Partner
(86) International application number: PCT/JP2005/004246
(87) International publication number: WO 2005/087268

(57) **Abstract**

An axon regeneration promoter is disclosed that contains an inhibitor of RGM-like protein as an effective component. Inhibitors of RGM-like protein encompass inhibitors of RGM-like protein such as anti-RGM-like protein antibodies and Y27632, as well as antisense nucleic acids and double-stranded RNAs that can inhibit the transcription or translation of RGM-like protein. The axon regeneration promoter according to the present invention is effective for the regeneration of central nerve axons and thus contributes, for example, to the treatment of patients who have suffered damage to the central nervous system, for example, the spinal cord, due to, for example, a traffic accident or a cerebrovascular disorder.

## Description

### TECHNICAL FIELD

The present invention relates to an axon regeneration promoter that can promote the regeneration of neuronal axons and particularly neuronal axons in the central nervous system.

### BACKGROUND ART

When central nerves, for example, the spinal cord, are injured due to a traffic accident, or are damaged by a cerebrovascular disorder, the neural function is lost and cannot be regenerated. This stands in direct contrast to the fact that peripheral nerves undergo regeneration. Damage to central nerves frequently results in partial or complete paralysis because central nerves, once damaged, cannot regenerate. Inducing regeneration of damaged central nerves is therefore an important issue in the field of medical care.

Axons of adult central nerves can regenerate through peripheral nerve grafts (S. David, A. J. Aguayo, Science 214, 931-3 (Nov 20, 1981)). This fact suggests that the major cause of the lack of regeneration in the adult central nerve is the local environment surrounding the neuron. To date, Nogo, myelin-associated glycoprotein (MAG), and oligodendrocyte-myelin glycoprotein (OMgp) have been identified as three main inhibitors of central nerve regeneration. Nogo was identified as the antigen corresponding to monoclonal antibody IN-1 (M. S. Chen et al., Nature 403, 434-9 (Jan 27, 2000); T. GrandPre, F. Nakamura, T. Vartanian, S. M. Strittmatter, Nature 403, 439-44 (Jan 27, 2000); P. Caroni, M. E. Schwab, Neuron 1, 85-96 (Mar, 1988)). MAG is known to play an important role in the formation and maintenance of the myelin sheath (S. Carenini, D. Montag, H. Cremer, M. Schachner, R. Martini, Cell Tissue Res 287, 3-9 (Jan, 1997); M. Fruttiger, D. Montag, M. Schachner, R. Martini, Eur J Neurosci 7, 511-5 (Mar 1, 1995); N. Fujita et al., J Neurosci 18, 1970-8 (Mar 15, 1998); J. Marcus, J. L. Dupree, B. Popko, J Cell Biol 156, 567-77 (Feb 4, 2002)) and has been found to inhibit axonal growth from certain neurons (G. Mukhopadhyay, P. Doherty, F. S. Walsh, P. R. Crocker, M. T. Filbin, Neuron 13, 757-67 (Sep, 1994); L. McKerracher et al., Neuron 13, 805-11 (Oct, 1994)). OMgp, which is the main peanut agglutinin-binding polypeptide in the white matter of adult central nerves (D. D. Mikol, K. Stefansson, J Cell Biol 106, 1273-9 (Apr, 1988)), has been identified as a third inhibitor of axonal growth (V. Kottis et al., J Neurochem 82, 1566-9 (Sep, 2002); K. C. Wang et al., Nature 417, 941-4 (Jun 27, 2002)). It is known that Nogo, MAG, and OMgp bind to NgR with p75 as coreceptor, suggesting that they have common signal transduction pathways (K. C. Wang, J. A. Kim, R. Sivasankaran, R. Segal, Z. He, Nature 420, 74-8 (Nov 7, 2002); M. Domeniconi et al., Neuron 35, 283-90 (Jul 18, 2002); A. E. Fournier, T. GrandPre, S. M. Strittmatter, Nature 409, 341-6 (Jan 18, 2001); T. Yamashita, H. Higuchi, M. Tohyama, J Cell Biol 157, 565-70 (May 13, 2002)).

Axonal regeneration through elimination or inhibition of these inhibitors has been investigated. However, it has been found through investigations using knockout mice for each of these inhibitors that central nerve axonal regeneration does not occur upon inhibitor elimination alone (U. Bartsch et al., Neuron 15, 1375-81 (Dec, 1995); C. J. Woolf, Neuron 38, 153-6 (Apr 24, 2003); J. E. Kim, S. Li, T. GrandPre, D. Qiu, S. M. Strittmatter, Neuron 38, 187-99 (Apr 24, 2003); B. Zheng et al., Neuron 38, 213-24 (Apr 24, 2003); M. Simonen et al., Neuron 38, 201-11 (Apr 24, 2003)). Furthermore, it has been reported that regeneration of the injured spinal cord was not promoted by depletion of functional p75NTR or by administration of soluble p75N-Fc (X. Song et al., J Neurosci 24, 542-6 (Jan 14, 2004)).

### References

[Nonpatent Reference 1] S. David, A. J. Aguayo, Science 214, 931-3 (Nov 20, 1981).
[Nonpatent Reference 2] M. S. Chen et al., Nature 403, 434-9 (Jan 27, 2000).
[Nonpatent Reference 3] T. GrandPre, F. Nakamura, T. Vartanian, S. M. Strittmatter, Nature 403, 439-44 (Jan 27, 2000). [Nonpatent Reference 4] P. Caroni, M. E. Schwab, Neuron 1, 85-96 (Mar, 1988).
[Nonpatent Reference 5] S. Carenini, D. Montag, H. Cremer, M. Schachner, R. Martini, Cell Tissue Res 287, 3-9 (Jan, 1997). [Nonpatent Reference 6] M. Fruttiger, D. Montag, M. Schachner, R. Martini, Eur J Neurosci 7, 511-5 (Mar 1, 1995).
[Nonpatent Reference 7] N. Fujita et al., J Neurosci 18, 1970-8 (Mar 15, 1998).
[Nonpatent Reference 8] J. Marcus, J. L. Dupree, B. Popko, J Cell Biol 156, 567-77 (Feb 4, 2002).
[Nonpatent Reference 9] G. Mukhopadhyay, P. Doherty, F. S. Walsh, P. R. Crocker, M. T. Filbin, Neuron 13, 757-67 (Sep, 1994).
[Nonpatent Reference 10] L. McKerracher et al., Neuron 13, 805-11 (Oct, 1994).
[Nonpatent Reference 11] D. D. Mikol, K. Stefansson, J Cell Biol 106, 1273-9 (Apr, 1988).
[Nonpatent Reference 12] V. Kottis et al., J Neurochem 82, 1566-9 (Sep, 2002).
[Nonpatent Reference 13] K. C. Wang et al., Nature 417, 941-4 (Jun 27, 2002).
[Nonpatent Reference 14] K. C. Wang, J. A. Kim, R. Sivasankaran, R. Segal, Z. He, Nature 420, 74-8 (Nov 7, 2002). [Nonpatent Reference 15] M. Domeniconi et al., Neuron 35, 283-90 (Jul 18, 2002).
[Nonpatent Reference 16] A. E. Fournier, T. GrandPre, S. M. Strittmatter, Nature 409, 341-6 (Jan 18, 2001).
[Nonpatent Reference 17] T. Yamashita, H. Higuchi, M. Tohyama, J Cell Biol 157, 565-70 (May 13, 2002).
[Nonpatent Reference 18] U. Bartsch et al., Neuron 15, 1375-81 (Dec, 1995).
[Nonpatent Reference 19] C. J. Woolf, Neuron 38, 153-6 (Apr 24, 2003).
[Nonpatent Reference 20] J. E. Kim, S. Li, T. GrandPre, D. Qiu, S. M. Strittmatter, Neuron 38, 187-99 (Apr 24, 2003).
[Nonpatent Reference 21] B. Zheng et al., Neuron 38, 213-24 (Apr 24, 2003).
[Nonpatent Reference 22] M. Simonen et al., Neuron 38, 201-11 (Apr 24, 2003).
[Nonpatent Reference 23] X. Song et al., J Neurosci 24, 542-6 (Jan 14, 2004).
[Nonpatent Reference 24] P. P. Monnier et al., Nature 419, 392-5 (Sep 26, 2002).
[Nonpatent Reference 25] B. K. Muller, D. G. Jay, F. Bonhoeffer, Curr Biol 6, 1497-502 (Nov 1, 1996).
[Nonpatent Reference 26] C. J. Woolf, S. Bloechlinger, Science 297, 1132-4 (Aug 16, 2002).
[Nonpatent Reference 27] B. Niederost, T. Oertle, J. Fritsche, R. A. McKinney, C. E. Bandtlow, J Neurosci 22, 10368-76 (Dec 1, 2002).
[Nonpatent Reference 28] M. Lehmann et al., J Neurosci 19, 7537-47 (Sep 1, 1999).
[Nonpatent Reference 29] P. Dergham et al., J Neurosci 22, 6570-7 (Aug 1, 2002).
[Nonpatent Reference 30] P. P. Monnier, A. Sierra, J. M. Schwab, S. Henke-Fahle, B. K. Mueller, Mol Cell Neurosci 22, 319-30 (Mar, 2003).
[Nonpatent Reference 32] A. A. Habib et al., J Neurochem 70, 1704-11 (Apr, 1998).
[Nonpatent Reference 33] H. Liu, C. E. Ng, B. L. Tang, Neurosci Lett 328, 257-60 (Aug 16, 2002).
[Nonpatent Reference 34] A. B. Huber, O. Weinmann, C. Brosamle, T. Oertle, M. E. Schwab, J Neurosci 22, 3553-67 (May 1, 2002). [Nonpatent Reference 35] D. Hunt, R. S. Coffin, P. N. Anderson, J Neurocytol 31, 93-120 (Feb, 2002).
[Nonpatent Reference 36] L. L. Jones, Y. Yamaguchi, W. B. Stallcup, M. H. Tuszynski, J Neurosci 22, 2792-803 (Apr 1, 2002).
[Nonpatent Reference 37] J. W. Fawcett, R. A. Asher, Brain Res Bull 49, 377-91 (Aug, 1999).

### DISCLOSURE OF THE INVENTION

An object of the present invention is to provide a novel axon regeneration promoter that can promote the regeneration of central nerve axons.

As a result of intensive investigations, the present inventors discovered that a protein exhibiting homology with the repulsive guidance molecule (RGM) that participates in the formation of the retinotectal projection (P. P. Monnier et al., Nature 419, 392-5 (Sep 26, 2002); B. K. Muller, D. G. Jay, F. Bonhoeffer, Curr Biol 6, 1497-502 (Nov 1, 1996)), is expressed in the white matter and grey matter of the spinal cord in the chick embryo (this protein is called "RGM-like protein" in this Specification and the attendant Claims) and that the expression of this protein increases when the spinal cord is damaged. It was further discovered that this protein has an inhibitory activity on the growth of central nerve axons. In addition, the present inventors discovered that the axonal growth-inhibiting activity of RGM-like protein is extinguished by treatment of RGM-like protein with an inhibitor of RGM-like protein, such as anti-RGM-like protein antibody or Y27632, and conceived of the utilization of inhibitors of RGM-like protein as axonal regeneration promoters, thus achieving the present invention.

The present invention provides an axon regeneration promoter containing an inhibitor of RGM-like protein as an effective component. This RGM-like protein inhibitor is preferably an anti-RGM-like protein antibody. Also preferably, the RGM-like protein inhibitor is Y27632.

The axons are preferably central nervous system axons.

In another aspect of the present invention, the present invention provides a method of identifying candidate substances for axon regeneration promoters, comprising a step of bringing a test substance into contact with RGM-like protein and determining whether the test substance inhibits the function of RGM-like protein.

The present invention provides a novel axon regeneration promoter that can promote the regeneration of central nerve axons. The axon regeneration promoter according to the present invention is effective for the regeneration of central nerve axons and is therefore expected to make a substantial contribution, for example, to the treatment of patients who have suffered damage to the central nervous system, such as the spinal cord.

### BRIEF DESCRIPTION OF THE DRAWINGS

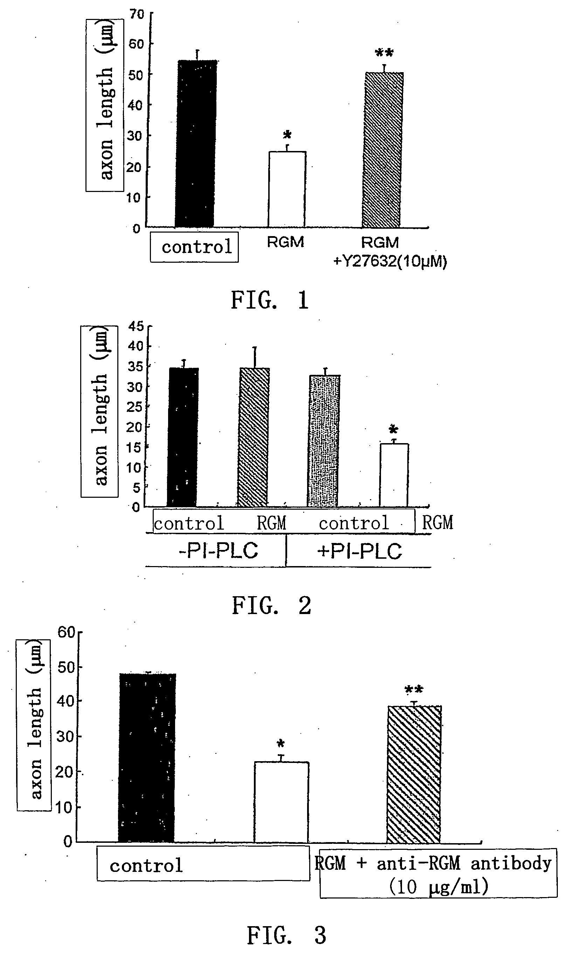

Figure 1 shows the results of axon growth assays carried out by plating cerebellar granule neurons on a confluent monolayer of rat RGM-like protein-expressing CHO cells or control CHO cells. The y-axis shows the average length of the longest axon of each neuron. The data is reported as the average ± standard deviation of three experiments. The single asterisk (*) indicates p < 0.01 relative to the control, while two asterisks (**) indicates p < 0.01 relative to RGM (Student's test).
Figure 2 shows the axon growth of neurons treated with various conditioned media. The y-axis shows the average length of the longest axon for each neuron. The data is reported as the average ± standard deviation of three experiments. The single asterisk (*) indicates p < 0.01 with respect to the axon length of neurons cultured in a conditioned medium from control CHO cells treated with PI-PLC.
Figure 3 shows the results of axon growth assays for cerebellar granule neurons cultured in media containing RGM-like protein, with and without the addition of anti-RGM-like protein polyclonal antibody. The y-axis shows the average length of the longest axon for each neuron. The data is reported as the average ± standard deviation of three experiments. The single asterisk (*) indicates p < 0.01 with respect to the control; double asterisks (**) indicate p < 0.01 with respect to RGM (Student's test). There was no significant difference between the control and RGM plus anti-RGM.

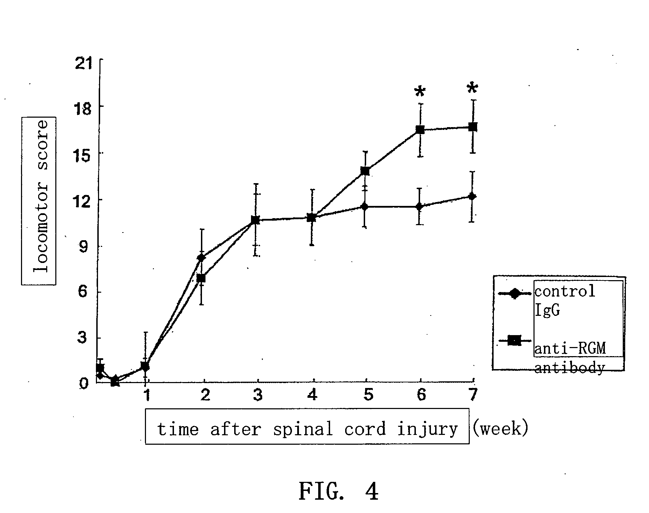

Figure 4 shows the BBB score after the elapsed time indicated in the figure after midthoracic dorsal hemisection, for rats receiving anti-RGM-like protein antibody and rats receiving control antibody.

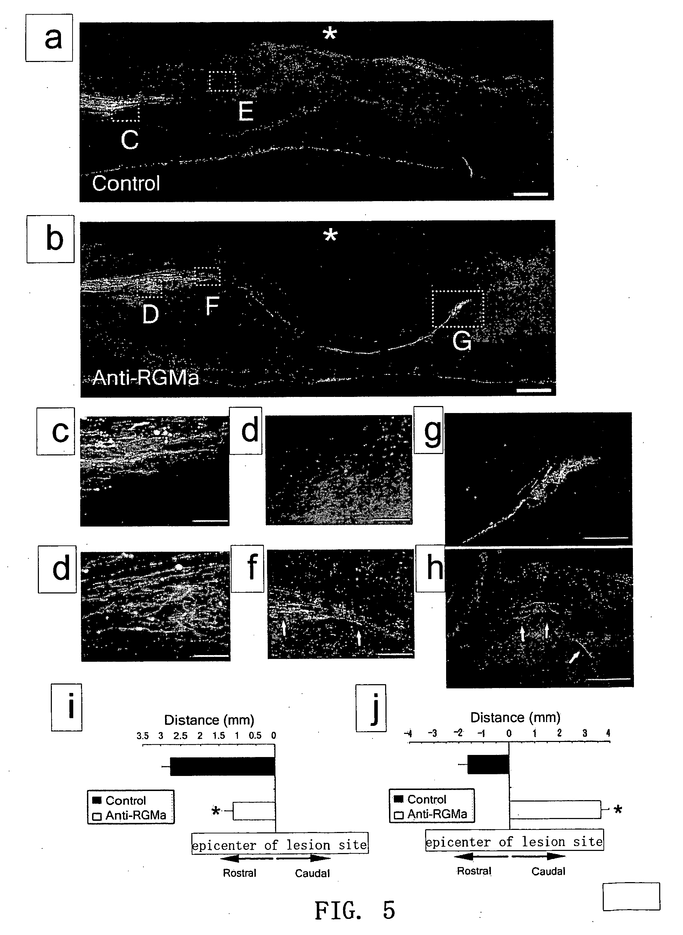

Figure 5 shows photomicrographs of the spine and the distance from the lesion epicenter of regenerating corticospinal (CST) fibers, for rats subjected to a midthoracic dorsal hemisection and treated with anti-RGM-like protein antibody or control antibody.

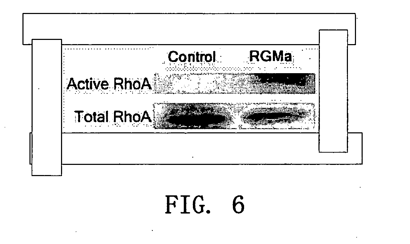

Figure 6 shows the Western blot results for measurement of the active Rho in rat cerebellar neurons treated with RGM-like protein.

### PREFERRED EMBODIMENTS OF THE INVENTION

As described above, the present inventors have previously discovered that RGM-like protein is expressed in the chick embryo in the white matter and grey matter of the spinal cord. It was observed that a gene having homology with chick embryo RGM-like protein is expressed in humans in the brain. The nucleotide sequence of the gene for human RGM-like protein and the amino acid sequence encoded by the gene are shown in SEQ ID NOs: 1 and 2. The nucleotide sequence of the gene for rat RGM-like protein and the amino acid sequence encoded by the gene are shown in SEQ ID NOs: 3 and 4.

As is specifically described in the examples provided below, RGM-like protein has an inhibitory activity on axonal growth and its expression increases when the spinal cord is damaged. Furthermore, it was found in the examples provided below that axon growth inhibition due to RGM-like protein is eliminated by the action of anti-RGM-like protein antibody on neurons. Accordingly, substances that neutralize the inhibitory activity of RGM-like protein on axon growth, such as anti-RGM-like protein antibody, can be used as axon regeneration promoters.

The axon regeneration promoter according to the present invention has an RGM-like protein inhibitor as an effective component. As used herein, the term "RGM-like protein inhibitor" denotes a substance that abolishes or at least significantly reduces the inhibitory activity exercised by RGM-like protein on axon regeneration. The inhibitory activity of RGM-like protein on axon growth can be examined as described in the examples provided below.

Anti-RGM-like protein antibody is a particularly preferred RGM-like protein inhibitor according to the present invention. As used herein, "anti-RGM-like protein antibody" denotes antibody that can bind RGM-like protein by an antigen-antibody reaction. The antibody may be a monoclonal antibody or a polyclonal antibody.

Polyclonal antibody that binds RGM-like protein can be obtained in accordance with methods that are well known in the art, from the serum of animals immunized with RGM-like protein as the sensitizing antigen. Monoclonal antibody that binds RGM-like protein can be obtained in accordance with methods that are well known in the art, by immunizing an animal using RGM-like protein as the sensitizing antigen; collecting the resulting immunocytes and fusing them with myeloma cells; cloning the antibody-producing hybridoma; and culturing this hybridoma.

In addition to antibodies produced by hybridomas, monoclonal antibody according to the present invention may also include genetically recombinant antibodies produced by a transformant that has been transformed by an expression vector containing antibody genes, chimeric antibodies, CDR-grafted antibodies, and fragments of these antibodies.

A genetically recombinant antibody can be prepared by cloning the antibody-encoding cDNA from a hybridoma that produces anti-RGM-like protein antibody; inserting the cDNA into an expression vector; transforming animal or plant cells with the vector; and culturing the resulting transformant. A chimeric antibody is an antibody composed of the heavy and light chain variable regions of an antibody originating from a certain animal and the heavy and light chain constant regions of an antibody originating from another animal. Fab, F(ab')2, Fab', scFv, and diabody are examples of antibody fragments capable of binding RGM-like protein.

### Preparation of antibody-producing cells

Cells that produce antibody that binds RGM-like protein can be obtained by methods that are well known in the art. An animal is immunized using RGM-like protein as the sensitizing antigen; the resulting immunocytes are collected and fused with myeloma cells; and the antibody-producing hybridoma is cloned. More specifically, RGM-like protein, the antigen, is first produced. Since the cDNA for RGM-like protein is present in commercially available brain cDNA libraries, an amplified product for this gene can be readily obtained by PCR templated on a commercially available brain cDNA library; the gene is then inserted into a suitable vector and RGM-like protein can be recombinantly produced. The RGM-like protein produced in this manner is then subcutaneously, intravenously, or intraperitoneally injected as antigen into an animal. For example, a mammal such as mouse, rat, hamster, rabbit, or goat can be used as the immunized animal. The antigen is preferably administered bound to a carrier protein or in combination with a suitable adjuvant such as Freund's complete adjuvant. A partial peptide from RGM-like protein may also be used as the antigen.

As shown in the examples provided below, it was discovered that, when using as immunogen a chemically synthesized partial peptide from amino acid residue 309 (the "309aa" nomenclature is used herein) to 322aa of rat RGM-like protein with the amino acid sequence shown in SEQ ID NO: 4, the polyclonal antibody raised against this partial peptide exercises a neutralizing activity on the inhibitory activity of RGM-like protein on axonal growth. Accordingly, a neutralizing antibody against human RGM-like protein can be obtained using as immunogen a human gene-based polypeptide containing the corresponding region.

The antigen is administered every 1 to 3 weeks for a plurality of times. The antibody titer is monitored by measuring the amount of immunoglobulin in the serum by, for example, ELISA. Once the antibody titer has been raised to an acceptable level, immunocytes are collected from the animal. Spleen cells are preferably used as the immunocytes. The antibody-producing immunocytes are fused with myeloma cells originating from the same species of mammal to produce hybridomas. A variety of myeloma cell strains used for hybridoma production are available commercially. Fusion can be carried out by methods that are well known in the art, for example, in the presence of PEG, and the fused cells are selected on an HAT medium. Hybridomas that produce antigen-binding antibody are selected by assaying the culture supernatant by, for example, ELISA. Hybridomas that produce the desired antibody can be cloned by the limiting dilution method. By carrying out the axonal growth assay described in the examples, infra, on the monoclonal antibodies produced by the resulting hybridomas, hybridomas can be selected that exhibit a neutralizing activity on the axonal growth inhibitory activity of RGM-like protein.

### Production of monoclonal antibodies

Monoclonal antibodies can be produced from the supernatant from the culture liquid obtained by culturing cells that produce monoclonal antibodies. Monoclonal antibodies can also be produced by the intraperitoneal administration of monoclonal antibody-producing cells into mice that have been treated beforehand with 2,4,10,14-tetramethylpentadecane; the mouse ascites fluid is then collected on day 7 to day 10 post-inoculation followed by centrifugal separation and collection of the supernatant. Purification of the monoclonal antibody can be carried out by the usual methods of protein purification, for example, procedures such as salting out, ultrafiltration, gel filtration, ion-exchange chromatography, affinity chromatography, HPLC, and so forth. Affinity chromatography on a protein A column is preferably carried out. The subclass of the purified monoclonal antibody can be determined by typing using a commercially available mouse monoclonal antibody isotyping kit.

### Analysis of antibody gene nucleotide sequences

The nucleotide sequence of an antibody gene encoding the monoclonal antibody according to the present invention can be obtained by analyzing the gene of the resulting monoclonal antibody-producing cell. The total RNA is extracted from the monoclonal antibody-producing cell and cDNA fragment is generated with reverse transcriptase using the RNA as a template. The V-region of the antibody gene is amplified by PCR using suitably designed primers, and the nucleotide sequence of the cDNA for the region is determined.

### Determination of antibody specificity

The binding specificity of the monoclonal antibody according to the present invention can be determined using methods known in the art, such as ELISA, RIA, immunothin-layer chromatography, BIAcore, or fluorescent antibody procedures. For example, monoclonal antibody according to the present invention is added in a twofold dilution series to a microplate on which RGM-like protein has been fixed; after incubation, an enzyme-labeled secondary antibody is added and substrate is then added to generate color; and the absorbance is measured using a microplate reader.

### Recombinant antibodies

A recombinant-type antibody can be produced using recombinant gene technology with a gene encoding the amino acid sequence of the antibody according to the present invention. To produce a recombinant-type anti-RGM-like protein monoclonal antibody, a gene encoding the antibody according to the present invention is incorporated into a suitable expression vector followed by introduction into a host cell. E. coli, yeast cells, mammalian cells, insect cells, and plant cells may be used as the host cell, and mammalian cells, for example, CHO, COS, and BHK, are particularly preferred. The vector can be introduced into the host cell by, for example, the calcium chloride method, calcium phosphate method, DEAE dextran method, methods using DOTAP cationic liposomes (Boehringer Mannheim Corporation), electroporation methods, and lipofection. The resulting transformed host cell is cultured and recombinant-type antibody is produced by expression of the antibody.

### Chimeric antibodies

A chimeric antibody is an antibody composed of the heavy and light chain variable regions of an antibody originating from a particular animal (for example, mouse) and the heavy and light chain constant regions of an antibody originating from another animal (for example, human). Chimeric antibodies can be recombinantly produced by cloning cDNA that encodes the antibody heavy and light chain variable regions, from a hybridoma that produces a monoclonal antibody that binds RGM-like protein; cloning cDNA that encodes the heavy and light chain constant regions of antibody originating from another animal; combining these cDNAs and inserting into a suitable expression vector; and inducing expression in a host cell.

### CDR-grafted antibodies

A CDR-grafted antibody is an antibody in which the complementarity-determining region (CDR) of an antibody from a certain animal (for example, mouse) has been grafted into the complementarity-determining region of an antibody from a different animal (for example, human). Genes encoding a CDR-grafted antibody can be obtained as follows. Nucleotide sequence of the genes each encoding CDR1, 2, and 3 are designed based on the gene sequences of the heavy and light chain variable regions of an antibody cloned from a hybridoma that produces monoclonal antibody that binds RGM-like protein, and the sequences are substituted for the sequences of the corresponding CDR1, 2, and 3 in a vector containing genes coding for the heavy and light chain variable regions of an antibody originating in another animal. For example, synthesis can be carried out by PCR using a plurality of primers designed in such a manner that murine antibody CDR connects to a human antibody framework region. Alternatively, the complete sequence may be constructed using synthetic DNA. The CDR-grafted antibody can be recombinantly produced by inducing the expression of this expression vector in a suitable host cell.

### Antibody fragments

Antibody fragments that can bind RGM-like protein, for example, Fab, F(ab')2, Fab', scFv, and diabody, can be produced by treating anti-RGM-like protein monoclonal antibody according to the present invention with an enzyme such as papain or trypsin. They can also be produced by introducing into a host cell an expression vector incorporating genes coding for such an antibody fragment to obtain a transformant that produces an antibody fragment.

Another example of an RGM-like protein inhibitor is Y27632 (M. Uehata et al., Nature 389, 990-4 (Oct 30, 1997)), which is known as an inhibitor of the serine/threonine kinase, Rho kinase. It was found, as shown in the examples provided below, that Y27632 exhibits an activity that neutralizes the axonal growth inhibitory activity of RGM-like protein. Y27632 has the following chemical structure.

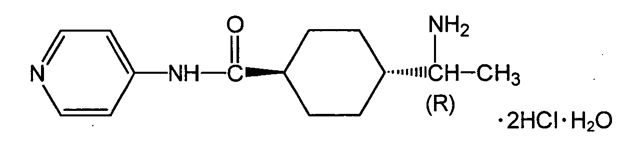

The axon regeneration promoter according to the present invention also includes, an inhibitor of RGM-like protein, antisense oligonucleotides, ribozymes, and molecules that cause RNA interference (RNAi) (for example, dsRNA, siRNA, shRNA, miRNA). These nucleic acids bind to the gene or mRNA coding for RGM-like protein and can thereby inhibit the expression thereof. General methods for inhibiting gene expression using antisense technology, ribozyme technology, and RNAi technology and gene therapy procedures that induce exogenous gene expression using these technologies are well known in the art.

An antisense oligonucleotide is a nucleic acid molecule or derivative thereof that has a sequence complementary to the mRNA encoding RGM-like protein. Antisense oligonucleotide binds specifically to mRNA and will inhibit protein expression by inhibiting transcription and/or translation. Binding may be through Watson-Crick or Hoogsteentype base pair complementarity or by triplex formation.

A ribozyme is a catalytic RNA structure of one or more RNAs. Ribozymes generally exhibit endonuclease, ligase, or polymerase activity. Ribozymes with various secondary structures are known, for example, hammerhead type ribozymes and hairpin type ribozymes. RNA interference (RNAi) refers to the technique of silencing a target gene using a double-stranded RNA molecule.

The RGM-like protein inhibitor can be administered as such, but is generally formulated using the carriers used for drugs. Any of the carriers ordinarily used in the formulation art can also be used as the carrier for this formulation; for example, physiological saline or phosphate-buffered physiological saline is preferably used for the preparation of an injectable. The usual additives, such as an emulsifying agent and an osmotic pressure regulator, may also be present.

The route of administration for the axon regeneration promoter according to the present invention is preferably a non-oral route, and direct injection at the site of the nerve damage is particularly preferred.

The dosage is selected as appropriate in correspondence to the type of RGM-like protein inhibitor, the route of administration, the degree of nerve damage, and so forth. However, in the case of direct administration to the site of damage, the adult dosage of RGM-like protein inhibitor per day per damage site is generally 1 to 20 mg and preferably 5 to 10 mg for anti-RGM-like protein antibody and is generally 20 to 100 mg and preferably 30 to 50 mg for Y27632. For non-oral administration other than direct administration, such as intravenous injection, the dosage is generally about 10 times the dosage cited above.

In another aspect, the present invention provides a method of identifying substances that are candidate axon regeneration promoters. This method comprises bringing a test substance into contact with RGM-like protein and determining whether the test substance inhibits the function of RGM-like protein. Inhibition of the function of RGM-like protein includes inhibition of the manifestation of the normal function of RGM-like protein through binding to RGM-like protein, and particularly inhibition of the capacity to promote axonal regeneration, as well as inhibition of binding by RGM-like protein to its receptor. The capacity of a test substance to bind to RGM-like protein, or the capacity of a test substance to inhibit binding by RGM-like protein to neogenin, its receptor, can be determined by binding assays that are well known to those skilled in the art. Nonlimiting examples are gel shift assays, radiolabeled competitive assays, and chromatographic fractionation. In addition, the test substance can be brought into contact with RGM-like protein in the presence of neogenin, the receptor for RGM-like protein, and the Rho activity can then be measured. Since Rho activity increases when RGM-like protein binds with neogenin, the absence of an increase in Rho activity in the presence of a test substance may indicate that the test substance inhibits the function of RGM-like protein.

A substance identified by these assays as inhibiting the function of RGM-like protein is considered to be a candidate axon regeneration promoter. Then, using an axon growth assay method known in the art, whether this candidate substance has an axon regeneration-promoting effect can be determined by measuring and comparing neuronal axon growth in the presence and absence of the candidate substance. A specific example of such an assay procedure is described in the examples provided below.

The contents of all the patents and reference articles expressly cited in the specification are incorporated herein by reference in its entirety. In addition, the contents of the description in the claims, specification and drawings of Japanese Patent Application Numbers 2004-68849 and 2004-273041, to which the instant application claims priority are also incorporated herein by reference in its entirety.

The present invention is described in greater detail with the examples provided below, but these examples do not limit the scope of the present invention.

### Example 1

### Cloning RGM-like protein

A BLAST search of the GenBank database was performed using the amino acid sequence of chicken RGM (P. P. Monnier et al., Nature 419, 392-5 (Sep 26, 2002)) as the query. Rat cDNA with accession no. XM_218791 (Rattus norvegicus) was identified as a result. The putative amino acid sequence showed 79% homology with chicken RGM protein and for this reason XM_218791 was designated rat RGM-like protein. Full-length rat RGM-like protein cDNA was isolated by PCR from an adult rat brain cDNA library. The nucleotide sequence of the forward primer used was agtggtaacaggccgagctggatgg (SEQ ID NO: 5); the nucleotide sequence of the reverse primer was ccacaaccttgtcgcgtgcactaat (SEQ ID NO: 6); PCR comprised 25 cycles of denaturation for 30 seconds at 95°C, annealing for 30 seconds at 55°C, and elongation for 3 minutes 30 seconds at 72°C. The encoded protein consisted of 431 amino acid residues. Native rat RGM was presumed to began at 152aa based on the previous report by Monnier et al., cited above. An HA-RGM vector was constructed in pSecTag2-Hygro (Invitrogen Corporation) using HA (hemagglutinin) and 152-431aa of the rat RGM-like protein with the signal peptide from pSecTag2 (Invitrogen Corporation). This procedure was specifically carried out as follows. Using full-length rat RGM-like protein cDNA as template, a BamHI-HAtag-(cDNA corresponding to 152-431aa of the RGM-like protein)-XhoI fragment was first constructed by PCR. The constructed fragment and pSecTag2-Hygro (Invitrogen Corporation) were cleaved by two restriction enzymes (BamHI and XhoI), followed by ligation of the two and transformation into E. coli. The sequence of entire PCR-amplified fragment was confirmed by DNA sequencing.

### Generation of RGM-like protein-CHO cells

Using the Flp-In System (TM, Invitrogen Corporation), RGM-like protein-expressing cells were generated according to the manufacturer's recommendations. An HA-RGM fragment containing signal peptide was generated from the pSecTag2 vector using two restriction enzymes (NheI and XhoI), and this fragment was ligated into pcDNA5FRT (Invitrogen Corporation). This construct (pcDNA5FRT/IgKleader/HA/RGM) and pOG44 were co-transfected into Flp-In CHO cells. The cells were grown for 2 weeks on medium containing hygromycin B (500 µg/mL, Invitrogen Corporation) to obtain cells stably expressing HA-RGM. HA-RGM expression was confirmed by Western blot and immunocytochemistry.

### Axon growth assays

Cerebellar granule cells from rat pups at 8 days postnatal (P8) were dissociated by trypsinization (0.25% trypsin in PBS, 37°C, 15 minutes); resuspended in serum-containing medium; triturated; and washed three times with PBS. For the coculture assay, neurons were plated onto a confluent monolayer of RGM-CHO cells or control CHO cells in chamber slides. 10 µM Y27632 (Welfide Corporation, Osaka) was added to the cultures to inhibit ROCK (Rho kinase). The cultures were grown for 24 hours in serum-free DMEM/F12 medium.

For the soluble RGM assay, confluent monolayers of RGM-CHO cells or control CHO cells were incubated in serum-free DMEM/F12 with or without 2.5 U/mL PI-PLC (phosphatidylinositol-specific phospholipase C, Sigma). After the culture were centrifuged for 10 minutes at 13000 g, floating cells were removed by collecting the supernatant. A portion of the supernatant was subjected to Western blot analysis. Neurons were plated on the conditioned media on poly-L-lysine-coated chamber slides and were incubated for 12 or 24 hours. For the axon assay, the cells were fixed with 4% (w/v) paraformaldehyde and immunostained with a monoclonal antibody that recognizes β-tubulin III protein specifically present in neurons (TuJ1, 1:1000, Covance Research Products, Inc., Denver, USA). The length of the longest neurite for each β-tubulin III-positive neuron was then measured.

### Spinal cord injury

Anesthetized (sodium pentobarbital, 40 mg/kg) female Wistar rats (200-250 g) received a laminectomy at vertebral level T10 and the spinal cord was exposed. A site of injury to the dorsal column, corticospinal tract, and a part of dorsal horn was created by cutting the spinal cord using a number 11 blade. The muscle and skin layers were then sutured. Until bladder function was recovered, the bladder was pressed at least twice a day by the application of pressure to the abdomen.

### Production of anti-rat RGM-like protein antibody

A partial peptide (309-322aa) of RGM-like protein was chemically synthesized and anti-rat RGM-like protein rabbit antiserum was obtained using the protein as an immunogen. The antiserum was subjected to affinity purification and was used for immunohistochemistry and immunoblotting at a concentration of 1 µg/mL and for the neutralizing antibody assay at a concentration of 10 µg/mL.

### Tissue preparation and immunohistochemistry

For immunohistochemistry, fresh frozen samples were obtained from an uninjured spinal cord and from spinal cords at 6 hours, 1 day, 3 days, and 7 days post-injury. Deep anesthesia with diethyl ether was followed by decapitation, after which the spinal cord was removed, embedded in Tissue Tek OCT (TM), and immediately frozen at -80°C on dry ice. A series of parasagittal sections was cut at the 18 µm position on a cryostat and mounted on APS coating Superfrost-Plus slides (TM, Matsunami Glass Ind., Ltd., Osaka). The sections were fixed for 1 hour at room temperature with 4% (w/v) paraformaldehyde, washed three times with PBS, and blocked for 1 hour at room temperature in PBS containing 5% goat serum (GS) and 0.1% Triton X-100 (TM). The sections were incubated overnight at 4°C with primary antibody, washed three times with PBS, and then incubated for 1 hour at room temperature with fluorescein-conjugated secondary antibody (1:1000, Molecular Probes). The following were used as the primary antibody: anti-rat RGM-like protein polyclonal antibody (1 µg/mL), anti-glial fibrillary acidic protein (GFAP) monoclonal antibody (1:1000, Sigma), anti- myelin/oligodendrocyte-specific protein (MOSP) monoclonal antibody (1:500, Chemicon International, Inc.), and monoclonal antibody that labeled β-tubulin III protein (TuJ1, 1:1000, Covance Research Products, Inc.). The samples were examined with a confocal scanning electron microscope (Carl Zeiss, Jena, Germany). To determine the specificity of the anti-rat RGM-like protein antibody, control and spinal cord injury (SCI) sections were stained in the presence of rat RGM-like protein-specific peptide (309-322aa). The addition of this peptide at 10 µg/mL resulted in a complete absence of tissue staining (data not shown).

### Neutralizing antibody assay

Cerebellar granule neurons were plated on poly-L-lysine-coated chamber slides in conditioned media derived by the PI-PLC treatment of control CHO cells or RGM-CHO cells. The anti-rat RGM-like protein antibody was added (10 µg/mL) to the conditioned media derived by the PI-PLC treatment of RGM-CHO cells. After incubation for 24 hours, a growth assay was carried out as described above.

### Western blot assay

Control CHO cells or RGM-CHO cells were lysed with 50 mM Tris-HCl pH 7.5, 150 mM NaCl, 10% glycerol, and 0.5% Brij-58 (Sigma) containing a protease inhibitor cocktail tablet (Roche Diagnostics, Mannheim, Germany). The cell lysates were cleared by centrifugation for 10 minutes at 4°C/13000 g; the supernatants were collected; and the protein concentration was normalized using a Bio-Rad DC protein assay kit (TM). Equal amounts of protein were boiled for 5 minutes in sample buffer containing 12% β-mercaptoethanol and subjected to SDS-PAGE. The conditioned media were treated in the same manner, except for the lysis buffer treatment. The protein was transferred to a PVDF membrane and was blotted for 1 hour at room temperature in PBS containing 5% skim milk and 0.05% Tween 20 (TM). The membrane was blotted overnight with anti-rat RGM-like protein polyclonal antibody (1 µg/mL) or anti-HA monoclonal antibody (1:1000, Sigma). The ECL chemiluminescence system (TM, Amersham Biosciences) and HRP (horseradish peroxidase)-conjugated secondary antibody (1:1000, Cell Signaling Technology, Inc.) were used for detection.

### Results

### The axon growth assays

The results are shown in Figure 1 for the axon growth assay carried out by plating cerebellar granule neurons on a confluent monolayer of rat RGM-like protein-expressing CHO cells or control CHO cells. As shown in Figure 1, the axon length for cerebellar granule neurons cultured on RGM-like protein-expressing CHO cells was significantly shorter than for the control. On the other hand, in the case of addition of Y27632, an axon growth promoter according to the present invention, to the medium, axon growth was about the same as for the control and was significant in comparison to culture on RGM-like protein-expressing CHO cells.

Next, it was examined whether RGM-like protein in solution form inhibits axonal growth. Prior to the test, it was confirmed by immunostaining using anti-HA antibody that HA-RGM-like protein is released into the medium by treatment of the medium with PI-PLC. GPI (glycosylphosphatidylinositol)-anchored proteins will be released from the membrane by treatment with PI-PLC. This experiment was carried out since it was conjectured that RGM-like protein, which is highly homologous with chicken RGM as cited above, might be a GPI-anchored protein. As a result, HA-RGM-like protein was detected only in media from PI-PLC-treated RGM-like protein-expressing CHO cells, while there was absolutely no detection of HA-RGM-like protein in media from control CHO cells and in media from PI-PLC-untreated RGM-like protein-expressing CHO cells. These observations confirmed that RGM-like protein is a GPI-anchored protein and is released into solution by PI-PLC treatment.

The axon length of conditioned medium-treated neurons is shown in Figure 2 for individual conditioned media. As shown in Figure 2, axon growth was significantly inhibited only for neurons treated by the medium obtained by PI-PLC treatment of RGM-like protein-expressing CHO cells. This result shows that RGM-like protein has an inhibitory action on axon growth. For media from the control CHO cells, neuron growth was not changed by the presence/absence of PI-PLC treatment, indicating that PI-PLC treatment itself does not influence axon growth.

### Distribution of RGM-like protein in the spinal cord

Prior to the tests, confirmatory tests were carried out as to whether the generated anti-RGM-like protein antibody exhibited specificity to the RGM-like protein. Using anti-HA antibody and the generated anti-RGM-like protein antibody, Western blotting was carried out on media from PI-PLC-treated RGM-like protein-expressing CHO cells. A band was detected at exactly the same position (approximately 35 kD) both for use of the anti-HA antibody and use of the generated anti-RGM-like protein antibody, while this band was not detected for the control. When the aforementioned peptide used as immunogen in generating the anti-RGM-like protein antibody was added at a concentration of 10 µg/mL to the anti-RGM-like protein antibody and used for immunohistostaining of fresh frozen sections of the normal spinal cord and damaged spinal cord of the adult rat, staining was completely absent. These observations confirmed that the generated anti-RGM-like protein antibody exhibits specificity to (i.e. undergoes an antigen-antibody reaction with) the RGM-like protein.

In order to examine the distribution of RGM-like protein, fresh frozen sections were prepared from the adult rat and were subjected to immunohistostaining. This immunohistostaining was specifically carried out as follows. The fresh frozen sections were first thoroughly dried at room temperature; then fixed for 1 hour with phosphate-buffered 4% paraformaldehyde solution and blocked for 1 hour; then treated with primary antibody overnight at 4°C; and thereafter treated with secondary antibody for 1 hour at room temperature. 0.1 M phosphate-buffered aqueous sodium chloride containing 10% goat serum and 0.1% Triton X was used as the blocking solution. The primary antibody solution was prepared by adding 1 µg/mL anti-RGM-like protein to this blocking solution. The secondary antibody solution was fluorescein-conjugated secondary antibody (1:1000, Molecular Probes) and 10% goat serum in 0.1 M phosphate-buffered aqueous sodium chloride. It was found that RGM-like protein was constitutively expressed in both the white matter and grey matter.

Double staining with anti-RGM-like protein and anti-MOSP (myelin/oligodendrocyte-specific protein) showed that RGM-like protein was expressed in oligodendrocytes and their processes in the white matter. In addition, RGM-like protein was localized to the somata of Tuj1 (neuron-specific β-tubulin III protein)-positive neurons, but was not localized to the axons of these cells in the white matter. When double immunohistostaining was carried out with anti rat-RGM-like protein and antibody against the astrocyte marker, GFAP (glial fibrillary acidic protein), regions in which these proteins were colocalized could not detected, demonstrating that RGM-like protein is not present in astrocytes. Collectively, RGM-like protein is expressed in neurons and oligodendrocytes and its expression pattern in the spinal cord is similar to that of Nogo and OMgp.

### RGM-like protein expression after spinal cord injury

RGM-like protein expression was detected at the epicenter of the lesion site and in the white matter rostral and caudal to the lesion site. In the epicenter region, a normal tissue structure was seen at 6 hours post-injury. When degenerative changes began to be observed at 1-3 days post-injury, immunoreactivity for RGM-like protein was also observed over the lesion site and in aberrant extracellular matrix. This extracellular immunoreactivity may be attributable to degeneration of the RGM-like protein-expressing cells. In order to characterize the RGM-like protein-expressing cells in the epicenter region, double-labeling experiments were carried out on the tissue at 7 days post-injury using anti-GFAP/anti-RGM-like protein, anti-MOSP/anti-RGM-like protein, and anti-Tuj1/anti-RGM-like protein. No double-labeled cells (i.e. GFAP positive and RGM-like protein positive; MOSP positive and RGM-like protein positive; or Tuj1 positive and RGM-like protein positive) were observed. Considering that damage to the central nervous system causes a glial scar, these cells are probably other types of cells that build the glial scar, for example, microglial cells, macrophages, oligodendrocyte precursors, fibroblasts, pial cells, and/or Schwann's cells.

In the white matter adjacent to the epicenter region, there were almost no changes up to 6 hours elapsed in the RGM-like protein-immunopositive cells and intensity of immunoreactivity. However, the intensity underwent a continuous increase at 1 to 3 days post-surgery. After 7 days, the signal density was significantly higher than for the uninjured spinal cord. Double-labeling experiments were carried out on tissue 7 days post-injury in order to characterize the RGM-like protein-expressing cells in the white matter. Double-labeled cells were not observed when double staining was carried out using anti-RGM-like protein/anti-GFAP and anti-RGM-like protein/anti-Tuj1. The results indicated that these cells were neither astrocytes nor neurons. Double-stained cells were detected when double staining for RGM-like protein and MOSP was carried out, demonstrating that RGM-like protein is strongly expressed in oligodendrocytes after spinal cord injury. Finally, it was confirmed that all the MOSP-expressing cells expressed RGM-like protein, while RGM-like protein-positive, MOSP-negative cells were not observed. It is thought that these cells in the white matter are the same as the cells observed in the epicenter of the lesion site. Accordingly, in response to spinal cord injury, the expression of RGM-like protein increases at the epicenter of the lesion site and in the white matter adjacent thereto.

### Neutralization by anti-RGM-like protein antibody of the inhibitory activity of RGM-like protein on axon growth

The results are shown in Figure 3. The single asterisk (*) in Figure 3 indicates that RGM is significantly shorter than the control. The double asterisk (**) indicates that RGM plus anti-RGM is significantly longer than for RGM. As shown in Figure 3, the inhibitory activity by RGM-like protein on axon growth was significantly neutralized by the addition of anti-RGM-like protein antibody, suggesting that anti-RGM-like protein polyclonal antibody can be used as an axon growth promoter.

### Example 2

### 1. Methods

### (1) Surgical procedure

Anesthetized (sodium pentobarbital, 40 mg/kg) female Wistar rats (200-250 g) received a laminectomy at vertebral level T9/10 and the spinal cord was exposed. The dorsal part of the spinal cord was cut to a depth of 1.8 mm using a number 11 blade. According to histologic examination, in all animals these lesions severed all the dorsal corticospinal tract (CST) fibers in the posterior column as well as the lateral corticospinal tract and extended past the central canal. This spinal cord hemisection was immediately followed by fitting with an osmotic pressure minipump (200 µL solution, 0.5 µL/hour, 14-day delivery, from Durect Corp., Cupertino, California, USA) filled with control antibody (8 animals, 22.3 µg/kg · day, 2 weeks) or the anti- RGM-like protein antibody generated in Example 1 (8 animals, 22.3 µg/kg · day, 2 weeks). The minipump was placed under the skin on the animal's back, and a silicone tube connected to the outlet of the minipump was placed under the dura mater at the spinal cord hemisection site so as to bring the tip immediately adjacent to the lesion site on the rostral side. The tube was fixed by suturing to the spinous process immediately caudal to the laminectomy site. The muscle and skin layers were then sutured. Until bladder function was recovered, the bladder was pressed at least twice a day by the manual application of pressure to the abdomen.

### (2) Tissue preparation and immunohistochemistry

Tissue preparation and immunohistochemistry were carried out as described in Example 1.

### (3) Anterograde labeling of the CST

Eight weeks after injury, biotin-dextran amine (BDA, 10% in physiological saline, 3.5 µL per cortex, molecular weight = 10,000, Molecular Probes, Eugene, Oregon, USA) was injected under anesthesia at the right and left motor cortices of the descending CST fibers (coordinates: 2 mm posterior to bregma, 2 mm lateral to bregma, 1.5 mm depth). In each injection, 0.25 µL BDA was injected over 30 seconds through a glass capillary tube with an internal diameter of 15-20 µm that was attached to a microliter syringe. In total, 6 control rats and 8 rats that received anti-RGM-like protein antibody after SCI were examined and compared. 14 days after BDA injection, the animals were killed by perfusion with PBS and then paraformaldehyde. Cryostat sections of the spinal cord across the lesion site were taken sagitally (50 µm). Transverse sections were taken rostral and caudal to the lesion site. The sections were blocked for 1 hour with PBS containing 0.5% bovine serum albumin (BSA) and were then incubated for 1 day with Alexa Fluor 488 (TM)-conjugated streptavidin (1:400, Molecular Probes) in PBS containing 0.15% BSA. The distance from the epicenter of the lesion site to the BDA-detected CST fiber that extended most caudally was measured.

### (4) Behavioral testing

The behavioral recovery was evaluated in an open field environment for 7 weeks after injury using the Basso-Beattie-Bresnahan (BBB) locomotor rating scale (Basso, D.M., Beattie, M.S., & Bresnahan, J.C., A sensitive and reliable locomotor rating scale for open field testing in rats. J Neurotrauma 12, 1-21 (1995)). Quantitation was carried out in a blinded manner.

### 2. Results

### (1) Functional improvement due to neutralization of RGM-like protein

Whether endogenous RGM-like protein functions as an inhibitor of axon regeneration in the injured central nervous system was evaluated as described above. The dorsal two-thirds of the spinal cord was extirpated in rats at vertebral level Th9/10, thereby extirpating the main and lateral corticospinal tracts. Anti-RGM-like protein neutralizing antibody or IgG as control was delivered by osmotic pressure minipump through a catheter placed subdurally near the thoracic injury site. There was no significant difference in lesion depth between the control group and treated group. The locomotor performance was monitored. The sham-operated rats, who lacked spinal cord damage, received full Basso-Beattie-Bresnahan (BBB) locomotor scores. All the rats were almost completely paraplegic one day after injury (Figure 4). Subsequently, a gradual and partial recovery of locomotor behavior as evaluated by the BBB score were observed. Up to week 4 after spinal cord injury there were no differences in BBB score between the control animals and animals receiving anti-RGM-like protein antibody. It is noteworthy that between 6 and 7 weeks after surgery the locomotor performance of the rats receiving anti-RGM-like protein antibody was significantly better than that of the control rats. Accordingly, anti-RGM-like protein antibody is effective for the treatment of rats with spinal cord injuries.

Figure 4 shows the BBB score after the elapsed time in the figure after midthoracic dorsal hemisection, for rats receiving anti-RGM-like protein antibody and rats receiving control antibody. The average ± standard deviation is shown for each group (6 or 8 rats). The single asterisk (*) indicates that the group receiving anti-RGM-like protein antibody was significantly different in that week from the control group. *: p < 0.05 compared with the control.

### (2) Promotion of axon regeneration by inhibition of RGM-like protein

The integrity of the dorsal CST (corticospinal tract) was investigated as described above in some previously tested rats by injecting BDA into the sensory-motor cortices. Samples from the injured rats treated with anti-RGM-like protein antibody (Figure 5b) showed a labeling pattern that was completely different from that in samples from control rats (Figure 5a). Proximal to the edge of the lesion site, the main CST stops rostral to the lesion in rats not treated with anti-RGM-like protein antibody (Figures 5a, c). This basic pattern reflects the fact that axon regeneration does not normally occur in the central nervous system. In contrast, numerous fibers sprouting from the labeled CST were observed in rats treated with anti-RGM-like protein antibody (Figures 5b, d). The sprouted axons extended in the grey matter to a greater extent than in the white matter. This picture of numerous fibers was observed in all of the injured animals that received the antibody. This result cannot be caused by differences in the degree of BDA uptake, because no difference was seen between the antibody-treated animals and the control animals for the total number of fibers in the dorsal CST rostral to the lesion site. When longitudinal sections across the lesion site were inspected for the two groups of rats, there was less CST fiber retraction and a larger number of collateral CST sprouts for the rats treated with the neutralizing antibody (Figures 5a, b, i). Regenerating fibers showing typical irregular meandering growth were frequently seen in tissue bridges at the level of the lesion site in the rats treated with anti-RGM-like protein (Figures 5d, f). These regenerating fibers grew into the dorsal white matter and the lesion scar and along cysts (Figures 5f, h). It is noteworthy that many axons crossed the lesion tissue and thus either grew around cavities or grew within the epicenter of the lesion. High-magnification microscopic observation of the CST fibers at the lesion site of neutralizing antibody-treated rats showed shapes that resembled a synapse-like swelling. On the other hand, BDA was not detected in the control rats (Figure 5e). In the antibody-treated rats, bundles of axons grew through the lesion tissue up to 3.5 mm, and there were also bundles of axons that extended as far as 5 mm caudal from the lesion site; numerous labeled fibers were observed (Figures 5g, h, j). On the other hand, fibers were not observed in the control rats. Many of these fibers went along branches and meanders rather than a linear trajectory. Numerous branches sprouting from the main tract were observed at the caudal-most part of the regenerating fibers. Accordingly, administration of anti-RGM-like protein antibody promotes regeneration of CST axons.

In Figure 5, a and b are representative photographs of BDA-labeled CST fibers, wherein the rostral side is shown on the left. Anterograde-labeled CST fibers are shown 10 weeks after injury for spinal cords treated with control IgG (a) or with anti-RGM-like protein antibody (b) (RGM-like protein is referred to as RGMa in Figures 5 and 6). The epicenter of the lesion site is indicated with an asterisk. Photographs c-g in Figure 5 are photographs taken at higher magnification of the regions delineated by the boxes in a and b of Figure 5. In rats treated with anti-RGM-like protein antibody, increased collateral CST fibers sprouting rostrally (d) and regenerating fibers in the lesion site (f, arrows) were seen, but these fibers were not observed in the corresponding regions for control IgG-treated rats (c, e). Photograph h contains a different section from the same animal as in b and shows regenerating fibers 2.6 mm caudally from the epicenter of the lesion site (arrows). The scale bar indicates 500 µm in a and b, 100 µm in c-f, and 200 µm in g and h. Figure 5i shows the distance of the BDA-detected main CST fibers from the lesion epicenter, 10 weeks after spinal cord injury for rats treated with anti-RGM-like protein antibody or IgG (n = 6-8/group). *: p < 0.01 compared with the control. The anti-RGM-like protein antibody shows an inhibition of retraction by the injured main CST. Figure 5j shows the distance from the lesion site epicenter of the most caudal BDA-detected CST fibers, 10 weeks after spinal cord injury for rats treated with anti-RGM-like protein antibody or IgG (n = 6-8/group). *: p < 0.01 compared with control.

### Example 3

### 1. Method

### (1) Affinity precipitation of GTP-RhoA

Cells were lysed in 50 mM Tris (pH 7.5) containing 1% Triton X-100 (TM), 0.5% sodium deoxycholate, 0.1% SDS, 500 mM NaCl, 10 mM MgCl₂, 10 µg/mL leupeptin, and 10 µg/mL aprotinin. The cell lysate was cleared by centrifugation at 4°C for 10 minutes × 13000 *g*, and the supernatant was incubated for 45 minutes at 4°C with 20 µg GST-Rho binding domain of Rhotekin beads (TM, Upstate Biotech). The beads were washed four times with washing buffer (50 mM Tris (pH 7.5) containing 1% Triton X-100 (TM), 150 mM NaCl, 10 mM MgCl₂, and 10 µg/mL each of leupeptin and aprotinin). Bound Rho was detected by Western blotting using monoclonal antibody against RhoA (Santa Cruz Biotech, Santa Cruz, California, USA).

### 2. Results

### (2) Affinity precipitation of GTP-RhoA

The RhoA activity in neurons was measured. Conditioned medium was added to cerebellar neurons from 7-day postnatal rats and the RhoA activity was measured after 30 minutes had elapsed (Figure 6). As shown in Figure 6, Rho activation was confirmed for neurons cultured on a medium derived by PI-PLC treatment of RGM-like protein-expressing CHO cells, as compared to neurons cultured on medium from control CHO cells. This result shows that RGM-like protein activates Rho.

### INDUSTRIAL APPLICABILITY

The axon regeneration promoter according to the present invention is effective for the regeneration of injured central nerves and is useful as a therapeutic agent for patients who have a damaged central nervous system.

## Claims

1. An axon regeneration promoter comprising an inhibitor of RGM-like protein as an effective component.

2. The axon regeneration promoter according to claim 1, wherein the inhibitor of RGM-like protein is an anti-RGM-like protein antibody.

3. The axon regeneration promoter according to claim 1, wherein the inhibitor of RGM-like protein is Y27632.

4. The axon regeneration promoter according to any one of claims 1 to 3, wherein the axon is an axon of the central nervous system.

5. A method of identifying a candidate substance for a axon regeneration promoter, comprising a step of bringing a test substance into contact with RGM-like protein, and determining whether the test substance inhibits the function of RGM-like protein.


SEQUENCE LISTING
<110>  BioClues Inc.

<120>  AXON REGENERATION PROMOTER

<130>  M2772 EP

<140>  05 72 0517.1
<141>  2005-03-10

<150>  JP 2004-68849
<151>  2004-03-11

<150>  JP 2004-273041
<151>  2004-09-21

<160>  6

<170>  PatentIn version 3.1

<210>  1
<211>  1299
<212>  DNA
<213>  homo sapiens

<400>  1
atggggagag gggcaggacg ttcagccctg ggattctggc cgaccctcgc cttccttctc      60
tgcagcttcc ccgcagccac ctccccgtgc aagatcctca agtgcaactc tgagttctgg     120
agcgccacgt cgggcagcca cgccccagcc tcagacgaca cccccgagtt ctgtgcagcc     180
ttgcgcagct acgccctgtg cacgcggcgg acggcccgca cctgccgggg tgacctggcc     240
taccactcgg ccgtccatgg catagaggac ctcatgagcc agcacaactg ctccaaggat     300
ggccccacct cgcagccacg cctgcgcacg ctcccaccgg ccggagacag ccaggagcgc     360
tcggacagcc ccgagatctg ccattacgag aagagctttc acaagcactc ggccaccccc     420
aactacacgc actgtggcct cttcggggac ccacacctca ggactttcac cgaccgcttc     480
cagacctgca aggtgcaggg cgcctggccg ctcatcgaca ataattacct gaacgtgcag     540
gtcaccaaca cgcctgtgct gcccggctca gcggccactg ccaccagcaa gctcaccatc     600
atcttcaaga acttccagga gtgtgtggac cagaaggtgt accaggctga gatggacgag     660
ctcccggccg ccttcgtgga tggctctaag aacggtgggg acaagcacgg ggccaacagc     720
ctgaagatca ctgagaaggt gtcaggccag cacgtggaga tccaggccaa gtacatcggc     780
accaccatcg tggtgcgcca ggtgggccgc tacctgacct ttgccgtccg catgccagag     840
gaagtggtca atgctgtgga ggactgggac agccagggtc tctacctctg cctgcggggc     900
tgccccctca accagcagat cgacttccag gccttccaca ccaatgctga gggcaccggt     960
gcccgcaggc tggcagccgc cagccctgca cccacagccc ccgagacctt cccatacgag    1020
acagccgtgg ccaagtgcaa ggagaagctg ccggtggagg acctgtacta ccaggcctgc    1080
gtcttcgacc tcctcaccac gggcgacgtg aacttcacac tggccgccta ctacgcgttg    1140
gaggatgtca agatgctcca ctccaacaaa gacaaactgc acctgtatga gaggactcgg    1200
gacctgccag gcagggcggc tgcggggctg cccctggccc cccggcccct cctgggcgcc    1260
ctcgtcccgc tcctggccct gctccctgtg ttctgctag                           1299

<210>  2
<211>  432
<212>  PRT
<213>  homo sapiens

<400>  2
   Met Gly Arg Gly Ala Gly Arg Ser Ala Leu Gly Phe Trp Pro Thr Leu
   1               5                   10                  15
   Ala Phe Leu Leu Cys Ser Phe Pro Ala Ala Thr Ser Pro Cys Lys Ile
               20                  25                  30
   Leu Lys Cys Asn Ser Glu Phe Trp Ser Ala Thr Ser Gly Ser His Ala
           35                  40                  45
   Pro Ala Ser Asp Asp Thr Pro Glu Phe Cys Ala Ala Leu Arg Ser Tyr
       50                  55                  60
   Ala Leu Cys Thr Arg Arg Thr Ala Arg Thr Cys Arg Gly Asp Leu Ala
   65 70 75 80
   Tyr His Ser Ala Val His Gly Ile Glu Asp Leu Met Ser Gln His Asn
   85 90 95
   Cys Ser Lys Asp Gly Pro Thr Ser Gln Pro Arg Leu Arg Thr Leu Pro
   100 105 110
   Pro Ala Gly Asp Ser Gln Glu Arg Ser Asp Ser Pro Glu Ile Cys His
   115 120 125
   Tyr Glu Lys Ser Phe His Lys His Ser Ala Thr Pro Asn Tyr Thr His
   130 135 140
   Cys Gly Leu Phe Gly Asp Pro His Leu Arg Thr Phe Thr Asp Arg Phe
   145 150 155 160
   Gln Thr Cys Lys Val Gln Gly Ala Trp Pro Leu Ile Asp Asn Asn Tyr
   165 170 175
   Leu Asn Val Gln Val Thr Asn Thr Pro Val Leu Pro Gly Ser Ala Ala
   180 185 190
   Thr Ala Thr Ser Lys Leu Thr Ile Ile Phe Lys Asn Phe Gln Glu Cys
   195 200 205
   Val Asp Gln Lys Val Tyr Gln Ala Glu Met Asp Glu Leu Pro Ala Ala
   210 215 220
   Phe Val Asp Gly Ser Lys Asn Gly Gly Asp Lys His Gly Ala Asn Ser
   225 230 235 240
   Leu Lys Ile Thr Glu Lys Val Ser Gly Gln His Val Glu Ile Gln Ala
   245 250 255
   Lys Tyr Ile Gly Thr Thr Ile Val Val Arg Gln Val Gly Arg Tyr Leu
   260 265 270
   Thr Phe Ala Val Arg Met Pro Glu Glu Val Val Asn Ala Val Glu Asp
   275 280 285
   Trp Asp Ser Gln Gly Leu Tyr Leu Cys Leu Arg Gly Cys Pro Leu Asn
   290 295 300
   Gln Gln Ile Asp Phe Gln Ala Phe His Thr Asn Ala Glu Gly Thr Gly
   305 310 315 320
   Ala Arg Arg Leu Ala Ala Ala Ser Pro Ala Pro Thr Ala Pro Glu Thr
   325 330 335
   Phe Pro Tyr Glu Thr Ala Val Ala Lys Cys Lys Glu Lys Leu Pro Val
   340 345 350
   Glu Asp Leu Tyr Tyr Gln Ala Cys Val Phe Asp Leu Leu Thr Thr Gly
   355 360 365
   Asp Val Asn Phe Thr Leu Ala Ala Tyr Tyr Ala Leu Glu Asp Val Lys
   370 375 380
   Met Leu His Ser Asn Lys Asp Lys Leu His Leu Tyr Glu Arg Thr Arg
   385 390 395 400
   Asp Leu Pro Gly Arg Ala Ala Ala Gly Leu Pro Leu Ala Pro Arg Pro
   405 410 415
   Leu Leu Gly Ala Leu Val Pro Leu Leu Ala Leu Leu Pro Val Phe Cys
   420 425 430

<210>  3
<211>  1296
<212>  DNA
<213>  Rattus norvegicus

<400>  3
atggggagag gggcaggacg ttcagccctg ggattgtggc cgaccctcgc cttccttctc      60
tgcagctttc ccgcagctat ctctccctgc aagatcctca agtgcaactc tgagttctgg     120
agcgccacgt cgtcaggcag ccacgcccct gcctctgacg acgtgcccga gttctgtgct     180
gccctgcgca cctacgccct gtgcacgcga cggacagccc gcacctgccg gggcgacctg     240
gcttaccact cggctgtcca tggcatagag gacctcatga gccagcacaa ctgctccaag     300
gatggcccca cctcacagcc tcgagtgcgc acgctcccgc cagctgggga cagccaggag     360
cgctcagata gccccgagat ctgccactat gagaagagtt tccacaagca ctcagctgcc     420
cccaactaca ctcactgcgg cctctttggg gacccacacc tcaggacttt cacagaccac     480
ttccagacat gtaaggtgca aggcgcttgg cctctcatcg acaataatta cctgaacgtg     540
caggtcacca atacacctgt gctgcccggc tctgccgcca ctgccaccag caagctcacc     600
atcatcttca agaacttcca agagtgtgtg gaccagaaag tataccaagc cgagatggac     660
gagcttccgt ccgcctttgc cgatggctcc aaaaacggtg gagataaaca cggagccaac     720

agcctgaaga tcacagagaa ggtgtcaggc cagcacgtgg agatccaggc caagtacatc 780
ggcaccacca tcgtggtgag acaggtgggc cgctacctga ccttcgccgt ccggatgccc 840
gaggaggtag tcaacgccgt ggaggaccgt gacagccaag gcctctacct ctgcctgcgg 900
ggctgcccgc tcaaccagca gatcgacttc caggctttcc gtgccaacgc cgagagccct 960
cgcaggccag cagctgccag cccctctcct gtggtccccg agacatttcc gtacgagaca 1020
gctgtggcca agtgcaaaga gaagctgcct gtagaagact tgtactacca ggcctgtgtc 1080
ttcgacctcc tcacgactgg cgacgtgaac ttcacgctgg ccgcctacta tgctttggag 1140
gatggcaaga tgctccactc caacaaggac aagctacacc tgtttgaaag gactcgggag 1200
ctgcctggcg ctgtggccgc tgcagcattt cccttggccc ccgagatgct cccgggcacc 1260
gtcacacttc tggtcctgct gcctctgttc tggtag 1296

<210> 4
<211> 431
<212> PRT
<213> Rattus norvegicus

<400> 4
Met Gly Arg Gly Ala Gly Arg Ser Ala Leu Gly Leu Trp Pro Thr Leu
1               5                   10                  15
Ala Phe Leu Leu Cys Ser Phe Pro Ala Ala Ile Ser Pro Cys Lys Ile
            20                  25                  30
Leu Lys Cys Asn Ser Glu Phe Trp Ser Ala Thr Ser Ser Gly Ser His
        35                  40                  45
Ala Pro Ala Ser Asp Asp Val Pro Glu Phe Cys Ala Ala Leu Arg Thr
    50                  55                  60
Tyr Ala Leu Cys Thr Arg Arg Thr Ala Arg Thr Cys Arg Gly Asp Leu
65                  70                  75                  80
Ala Tyr His Ser Ala Val His Gly Ile Glu Asp Leu Met Ser Gln His
                85                  90                  95
Asn Cys Ser Lys Asp Gly Pro Thr Ser Gln Pro Arg Val Arg Thr Leu
            100                 105                 110
Pro Pro Ala Gly Asp Ser Gln Glu Arg Ser Asp Ser Pro Glu Ile Cys
        115                 120                 125
His Tyr Glu Lys Ser Phe His Lys His Ser Ala Ala Pro Asn Tyr Thr
    130                 135                 140
His Cys Gly Leu Phe Gly Asp Pro His Leu Arg Thr Phe Thr Asp His
145                 150                 155                 160
Phe Gln Thr Cys Lys Val Gln Gly Ala Trp Pro Leu Ile Asp Asn Asn
                165                 170                 175
Tyr Leu Asn Val Gln Val Thr Asn Thr Pro Val Leu Pro Gly Ser Ala
            180                 185                 190
Ala Thr Ala Thr Ser Lys Leu Thr Ile Ile Phe Lys Asn Phe Gln Glu
        195                 200                 205
Cys Val Asp Gln Lys Val Tyr Gln Ala Glu Met Asp Glu Leu Pro Ser
    210                 215                 220
Ala Phe Ala Asp Gly Ser Lys Asn Gly Gly Asp Lys His Gly Ala Asn
225                 230                 235                 240
Ser Leu Lys Ile Thr Glu Lys Val Ser Gly Gln His Val Glu Ile Gln
                245                 250                 255
Ala Lys Tyr Ile Gly Thr Thr Ile Val Val Arg Gln Val Gly Arg Tyr
            260                 265                 270
Leu Thr Phe Ala Val Arg Met Pro Glu Glu Val Val Asn Ala Val Glu
        275                 280                 285
Asp Arg Asp Ser Gln Gly Leu Tyr Leu Cys Leu Arg Gly Cys Pro Leu
    290                 295                 300
Asn Gln Gln Ile Asp Phe Gln Ala Phe Arg Ala Asn Ala Glu Ser Pro
305                 310                 315                 320
Arg Arg Pro Ala Ala Ala Ser Pro Ser Pro Val Val Pro Glu Thr Phe
                325                 330                 335
Pro Tyr Glu Thr Ala Val Ala Lys Cys Lys Glu Lys Leu Pro Val Glu
            340                 345                 350
Asp Leu Tyr Tyr Gln Ala Cys Val Phe Asp Leu Leu Thr Thr Gly Asp
        355                 360                 365
Val Asn Phe Thr Leu Ala Ala Tyr Tyr Ala Leu Glu Asp Gly Lys Met

370                 375                 380
Leu His Ser Asn Lys Asp Lys Leu His Leu Phe Glu Arg Thr Arg Glu
385                 390                 395                 400
Leu Pro Gly Ala Val Ala Ala Ala Ala Phe Pro Leu Ala Pro Glu Met
                405                 410                 415
Leu Pro Gly Thr Val Thr Leu Leu Val Leu Leu Pro Leu Phe Trp
            420                 425                 430

<210>  5
<211>  25
<212>  DNA
<213>  homo sapiens

<400>  5
agtggtaaca ggccgagctg gatgg                                          25

<210>  6
<211>  25
<212>  DNA
<213>  homo sapiens

<400>  6
ccacaacctt gtcgcgtgca ctaat                                          25